# FASCICULE DE BREVET EUROPEEN

(11) **EP 1 513 833 B1**
(45) Date de publication et mention de la délivrance du brevet: **19.04.2006**
(21) Numéro de dépôt: 03750780.3
(22) Date de dépôt: 18.06.2003
(51) Int. Cl.: C07D 405/04, C07D 405/14, A61K 31/4709, A61P 35/00

(54) **NOUVEAUX DERIVES DE 3-(4-OXO-4H-CHROMEN-2-YL)-(1H)-QUINOLEIN-4-ONES, LEUR PROCEDE DE PREPARATION ET LES COMPOSITIONS PHARMACEUTIQUES QUI LES CONTIENNENT**
NEUE 3-(4-OXO-4H-CHROMEN-2-YL)-(1H)-QUINOLIN-4-ONE-DERIVATE; VERFAHREN ZU DEREN HERSTELLUNG UND PHARMAZEUTISCHE ZUSAMMENSETZUNGEN, DIE DIESE ENTHALTEN
NOVEL 3-(4-OXO-4H-CHROMEN-2-YL)-(1H)-QUINOLINE-4-ONE DERIVATIVES, METHOD FOR PREPARING SAME AND PHARMACEUTICAL COMPOSITIONS CONTAINING SAME

(30) Priorité: 19.06.2002 FR 0207536
(43) Date de publication de la demande: 16.03.2005
(73) Titulaire: Les Laboratoires Servier, 92415 Courbevoie Cedex (FR)
(72) Inventeur: BRION, Jean-Daniel, 95320 Saint Leu le Fôret (FR); ISRAEL, Lucien, 75001 Paris (FR); LE RIDANT, Alain, 92200 Neuilly sur Seine (FR); HARPEY, Catherine, 75016 Paris (FR); RABHI, Chérif, 93260 Les Lilas (FR); KALOUN, El Bachir, 95800 Cergy Saint Christophe (FR)
(86) Numéro de dépôt international: PCT/FR2003/001849
(87) Numéro de publication internationale: WO 2004/000834

(56) Documents cités:
- EP-A- 0 758 649
- CHEMICAL ABSTRACTS, vol. 123, no. 5, 1995 Columbus, Ohio, US; abstract no. 55705x, page 906; XP002232988 & JP 00 733743 A (KYORIN SEIYAKU) 3 février 1995 (1995-02-03)

## Description

La présente invention concerne de nouveaux dérivés de 3-(4-oxo-4H-chromén-2-yl)-(1H)-quinoléin-4-ones, leur procédé de préparation, les compositions pharmaceutiques qui les contiennent ainsi que leur utilisation en tant qu'anti-cancéreux.

Les besoins de la thérapeutique anticancéreuse exigent le développement constant de nouveaux agents antitumoraux, dans le but d'obtenir des médicaments à la fois plus actifs et mieux tolérés.

La demande de brevet EP 0 758 649 décrit des dérivés de chromone qui sont des inhibiteurs de la matrice métalloprotéinase. La demande de brevet JP 0 733 743 décrit des dérivés de 2-aryl-4-quinoline qui présentent une activité antitumorale. Ces composés arrêtent totalement la mitose des cellules cancéreuses en métaphase.

Les composés de l'invention, outre le fait qu'ils soient nouveaux, présentent des propriétés antitumorales très intéressantes.

Ils ont, d'une part, un effet pro-apoptotique, une efficacité indépendante de l'expression de p53, pRb et Bcl-2 et un effet anti-angiogénique marqué, et d'autre part, une synergie avec l'action d'un grand nombre d'agents thérapeutiques cytostatiques sans qu'existe aucune hématotoxicité additionnelle ni d'une façon générale des manifestations d'intolérance.

Ces propriétés font des composés de l'invention à la fois des adjuvants très efficaces et bien tolérés des chimiothérapies, et des agents susceptibles de maintenir et prolonger les effets de ces chimiothérapies lorsqu'elles sont suspendues pour différentes raisons d'intolérance, fin de cure, suspension en raison d'une chirurgie, etc.

De par leurs propriétés, les composés de l'invention peuvent être associés avantageusement à l'ensemble des traitements cytotoxiques actuellement en usage, mais aussi aux radiothérapies, dont ils n'augmentent pas la toxicité, et aux diverses hormonothérapies à visée anticancéreuses (sein et prostate).

Plus spécifiquement, la présente invention concerne les composés de formule (I) : 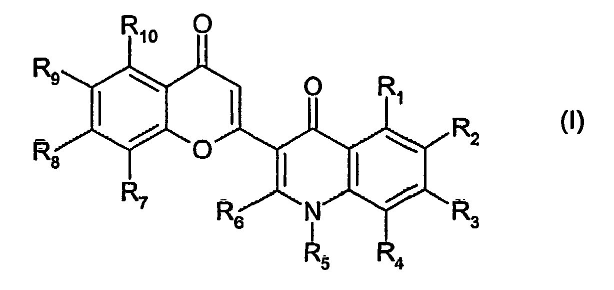 dans laquelle :
- R₁, R₂, R₃, R₄, R₆, R₈, R₉ et R₁₀, identiques ou différents, représentent chacun un groupement choisi parmi hydrogène, hydroxy, alkoxy (C₁-C₆) linéaire ou ramifié, alkyle (C₁-C₆) linéaire ou ramifié, arylalkoxy dans lequel le goupement alkoxy est (C₁-C₆) linéaire ou ramifié, alkoxycarbonylalkoxy dans lequel chacun des groupements alkoxy est (C₁-C₆) linéaire ou ramifié, et OR' dans lequel R' représente un groupement ionisé ou ionisable tel que, par exemple, un groupement phosphate -PO(OH)₂, sulfate -SO₃H, carboxyalkylcarbonyle dans lequel le goupement alkyle est (C₁-C₆) linéaire ou ramifié, dialkylaminoalkylcarbonyle dans lequel chacun des goupements alkyle est (C₁-C₆) linéaire ou ramifié, ou carboxyalkylaminocarbonyle, dans lequel le goupement alkyle est (C₁-C₆) linéaire ou ramifié,
- R₅ représente un groupement choisi parmi alkyle (C₁-C₆) linéaire ou ramifié, aryle et hétéroaryle,
- R₇ représente un groupement choisi parmi hydrogène, hydroxy, alkoxy (C₁-C₆) linéaire ou ramifié, alkyle (C₁-C₆) linéaire ou ramifié et cycloalkyle (C₃-C₇), ou bien R₇ représente un hétérocycle azoté ou oxygéné,
   leurs isomères optiques lorsqu'ils existent, leurs sels d'addition à un acide pharmaceutiquement acceptable ainsi que leurs hydrates et leurs solvates.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, oxalique, méthanesulfonique, benzènesulfonique, camphorique.

Par groupement aryle, on entend phényle, biphénylyle, naphtyle, ou tétrahydronaphtyle, chacun de ces groupements étant éventuellement substitué par un ou plusieurs atomes ou groupements, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, amino (substitué éventuellement par un ou deux groupements alkyle (C₁-C₆) linéaire ou ramifié), nitro, ou alkylènedioxy (C₁-C₂).

Par groupement hétéroaryle, on entend un groupement de 5 à 12 chaînons, soit monocyclique aromatique, soit bicyclique dont l'un au moins des cycles possède un caractère aromatique, et contenant un, deux ou trois hétéroatomes choisis parmi oxygène, azote ou soufre, étant entendu que l'hétéroaryle peut être éventuellement substitué par un ou plusieurs atomes ou groupements, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoallcyle (C₁-C₆) linéaire ou ramifié, ou amino (substitué éventuellement par un ou plusieurs groupements alkyle (C₁-C₆) linéaire ou ramifié). Parmi les groupements hétéroaryle, on peut citer à titre non limitatif les groupements thiényle, pyridyle, furyle, pyrrolyle, imidazolyle, oxazolyle, isoxazolyle, thiazolyle, isothiazolyle, quinolyle, isoquinolyle, pyrimidinyle.

Par hétérocycle azoté, on entend un groupement monocyclique, saturé ou insaturé, de 5 à 7 chaînons, contenant un atome d'azote, et éventuellement substitué par un ou plusieurs groupements choisis parmi hydroxy, alkoxy (C₁-C₆) linéaire ou ramifié, alkyle (C₁-C₆) linéaire ou ramifié, arylalkyle (C₁-C₆) linéaire ou ramifié, aminoalkyle (C₁-C₆) linéaire ou ramifié dans lequel le groupement amino est éventuellement substitué par un ou deux groupements alkyle (C₁-C₆) linéaire ou ramifié.

Les hétérocycles azotés préférés sont les groupements pipéridyle et tétrahydropyridyle éventuellement substitués.

Par hétérocycle oxygéné, on entend un groupement monocyclique, saturé ou insaturé, de 5 à 7 chaînons, contenant un atome d'oxygène, et éventuellement substitué par un ou plusieurs groupements choisis parmi hydroxy, alkoxy (C₁-C₆) linéaire ou ramifié, alkyle (C₁-C₆) linéaire ou ramifié, arylalkyle (C₁-C₆) linéaire ou ramifié, aminoalkyle (C₁-C₆) linéaire ou ramifié dans lequel le groupement amino est éventuellement substitué par un ou deux groupements alkyle (C₁-C₆) linéaire ou ramifié.

Un aspect avantageux de l'invention concerne les composés de formule (1) pour lesquels R₅ représente un groupement aryle.

Un autre aspect avantageux de l'invention concerne les composés de formule (1) pour lesquels R₇ représente un atome d'hydrogène.

Un autre aspect avantageux de l'invention concerne les composés de formule (I) pour lesquels R₇ représente un hétérocycle azoté éventuellement substitué.

Les composés préférés de formule (I) sont ceux pour lesquels R₅ représente un groupement phényle et R₇ représente un atome d'hydrogène ou un groupement 1,2,3,6-tétrahydro-4-pyridyle substitué.

Parmi les composés préférés de l'invention, on peut citer plus particulièrement :
- la 3-(5-hydroxy-4-oxo-4*H*-1-benzopyran-2-yl)-1-phényl-1*H*-quinoléin-4-one,
- la 3-[5,7-diméthoxy-8-(1-méthyl-1,2,5,6-tétrahydropyridin-4-yl)-4-oxo-4*H*-1-benzopyran-2-yl]-1-phényl-1*H*-1,4-dihydroquinoléin-4-one,
- la 3-(5,7-dihydroxy-4-oxo-4*H*-1-benzopyran-2-yl)-1-phényl-1*H*-1,4-dihydrôquinoléin-4-one,
- et la 3-[5,7-dihydroxy-8-(1-méthyl-1,2,5,6-tétrahydropyridin-4-yl)-4-oxo-4*H*-1-benzopyran-2-yl]-1-phényl-1*H*-1,4-dihydroquinoléin-4-one,

L'invention s'étend également à un procédé de préparation des composés de formule (I) caractérisé en ce que l'on met en réaction un composé de formule (II) : 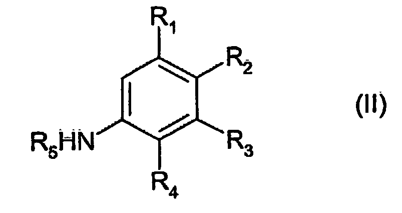 dans lequel R₁, R₂, R₃, R₄ et R₅ sont tels que définis dans la formule (I),
avec de l'éthoxyméthylène malonate de diéthyle, pour conduire au composé de formule (III) : 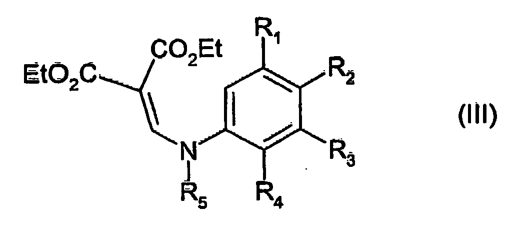 dans lequel R₁, R₂, R₃, R₄ et R₅ sont tels que définis précédemment, et Et représente le groupe éthyle,
que l'on cyclise dans des conditions acides, pour conduire au composé de formule (IV) : 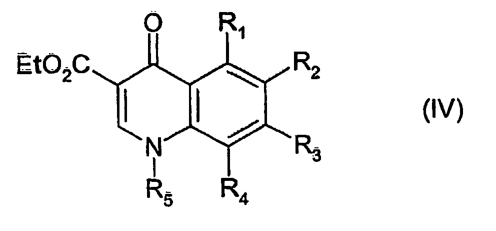 dans lequel R₁, R₂, R₃, R₄ et R₅ et Et sont tels que définis précédemment,
que l'on saponifie, pour conduire au composé de formule (V) : 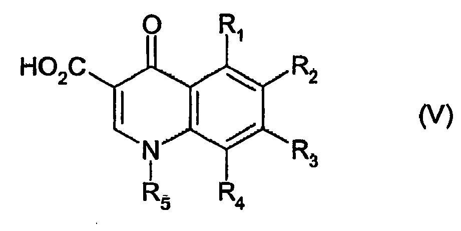 dans lequel R₁, R₂, R₃, R₄ et R₅ sont tels que définis précédemment,
que l'on transforme en chlorure d'acide par action de chlorure de thionyle, puis que l'on met en réaction avec le composé de formule (VI) : 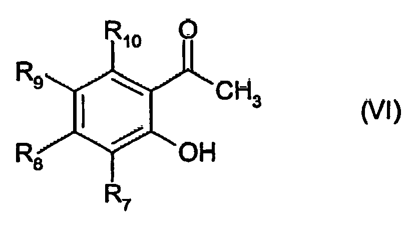 dans lequel R₇, R₈, R₉ et R₁₀ sont tels que définis dans la formule (I),
pour conduire au composé de formule (VII) : 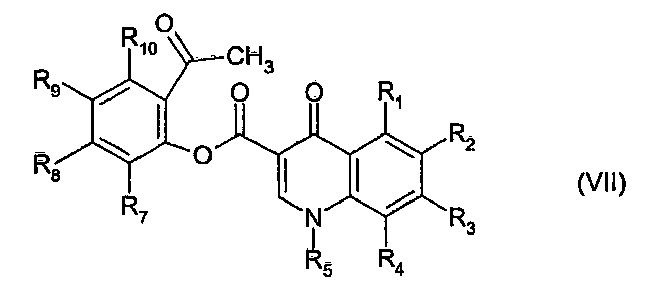 dans lequel R₁, R₂, R₃, R₄, R₅, R₆, R₇, R₈, R₉ et R₁₀ sont tels que définis précédemment,
que l'on soumet à l'action d'une base, pour conduire au composé de formule (VIII): 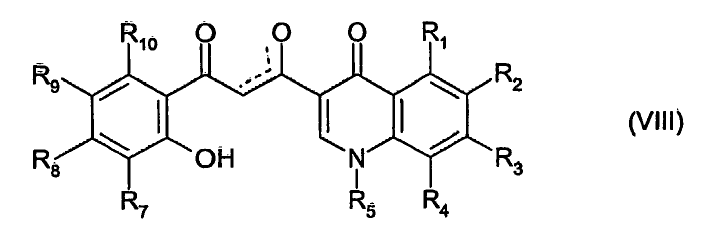 dans lequel R₁, R₂, R₃, R₄, R₅, R₆, R₇, R₈, R₉ et R₁₀ sont tels que définis précédemment, et 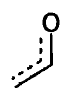 signifie que le composé est obtenu selon les molécules sous la forme d'un mélange céto-énolique,
que l'on soumet ensuite à des conditions acides pour conduire au composé de formule (I), que l'on purifie, le cas échéant, selon une technique classique de purification, dont on sépare, le cas échéant, les isomères optiques selon une technique classique de séparation, et que l'on transforme, si on le souhaite, en leurs sels d'addition à un acide pharmaceutiquement acceptable.

Les composés de formule (I) pour lesquels un ou plusieurs des substituants R₁ à R₄ et R₆ à R₁₀ représentent un groupement hydroxy, peuvent également être obtenus par clivage des composés de formule (I) pour lesquels le ou les substituants correspondants représentent un groupement alkoxy (C₁-C₆) linéaire ou ramifié.

Les composés de formule (I) pour lesquels un ou plusieurs des substituants R₁ à R₄ et R₆ à R₁₀ représentent un groupement alkoxycarbonylalkoxy, arylalkoxy ou OR', peuvent également être obtenus à partir des composés de formule (I) pour lesquels le ou les substituants correspondants représentent un groupement hydroxy.

Les composés de la présente invention, outre le fait qu'ils soient nouveaux, présentent des propriétés antitumorales très intéressantes, qui les rendent utiles dans le traitement des cancers.

Parmi les types de cancer que les composés de la présente invention peuvent traiter, on peut citer à titre non limitatif les adénocarcinomes, carcinomes, sarcomes, gliomes et leucémies.

Ils peuvent également être utilisés en association thérapeutique avec un autre anticancéreux tel que, par exemple, le paclitaxel, le tamoxinte et ses dérivés, le cisplatine et ses analogues, l'irinotécan et ses métabolites, les divers alkylants dont le chef de file est le cyclophosphamide, l'étoposide, les vincaalcaloïdes, la doxorubicine et autres anthracyclines, les nitrosouiées.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse, intramusculaire ou sous=cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient et les traitements éventuellement associés. Cette posologie varie de 0,5 mg à 2 g par 24 heures en une ou plusieurs prises.

Les exemples suivants illustrent l'invention et ne la limitent en aucune façon.

Les produits de départ utilisés sont des produits connus ou préparés selon des modes préparatoires connus.

Les structures des composés décrits dans les exemples ont été déterminées selon les techniques spectrométriques usuelles (infrarouge, RMN, spectrométrie de masse).

### EXEMPLE 1 : 3-(7-Méthoxy-4-oxo-4H-1-benzopyran-2-yl)-1-phényt-1H-1,4-dihydroquinoléin-4-one :

### Stade A : N-N-diphénylaminométhylènemalonate de diéthyle :

A 10 mmoles de diphénylamine sont ajoutées 10 mmoles d'éthoxyméthylènemalonate de diéthyle, puis le mélange est porté à 140-150 °C pendant 5 h. Après retour à température ambiante, le solide formé est rincé avec 100 mL d'éther diéthylique et recristallisé dans l'hexane pour conduire au produit attendu sous la forme d'un solide brun.
*Point de fusion :* 146-148 °C.
*SM* (IE, m/z) : 339,9 (M⁺).

### Stade B : 4-Oxo-1-phényl-1H-1,4-dihydroquinoléine-3-carboxylate d'éthyle :

A 10 mmoles du composé obtenu au stade précédent sont ajoutés 13,3 g d'acide polyphosphorique. Le mélange (qui devient progressivement liquide) est ensuite porté à 150-160 °C pendant 45 min, puis refroidi à 90 °C. Après hydrolyse, le milieu est neutralisé à l'aide d'une solution de NaOH à 10 % pour conduire après isolement au produit attendu.
*IR* (lames NaCl, cm⁻¹). 1733 (νC=O), 1610 (νC=C), 1645 (νC=O), 690 (νC-Har).
*SM* (IE, m/z) : 293,3 (M⁺).

### Stade C : Acide 4-oxo-1-phényl-1H-1,4-dihydroquinoléine-3-carboxylique:

A 10 mmoles du composé obtenu au stade précédent en solution dans le méthanol sont ajoutés 38 mL d'une solution de NaOH 2M. Le mélange réactionnel est ensuite porté au reflux du méthanol pendant 10 h, puis le solvant est éliminé sous vide. Au résidu obtenu est ajoutée de l'eau, puis le mélange est neutralisé par une solution de HCl 4M. Le solide gris obtenu est lavé à l'eau, puis séché pour conduire au produit attendu.
*Point de fusion:* 210-213 °C.
*IR* (KBr, cm⁻¹): 3320 (νOH acide), 1733 (νC=O), 1610 (νC=C), 1645 (νC=O), 690 (νC-Har):
*SM* (IE, m/z) : 265,3 (M⁺).

### Stade D : 4-Oxo-1-phényl-1H-1,4-dihydroquinoléiné-3-carboxylate de (2-acétyl-5-méthoxy)phényle:

A 20 mmoles de chlorure de thionyle en solution dans le dichloroéthanè, sont ajoutés, sous agitation, 10 mmoles du composé obtenu au stade précédent. Le milieu réactionnel est chauffé au reflux du solvant pendant 2 h, puis concentré sous vide et l'excès de chlorure de thionyle est éliminé par distillation sous vide avec entraînement à plusieurs reprises par du dichloroéthane.

Le chlorure d'acide ainsi obtenu (solide blanc) est ajouté par petites fractions à 6,7 mmoles de 2-hydroxy-4-méthoxyacétophénone commerciale en solution dans la pyridine. Après 12h d'agitation sous atmosphère inerte à température ambiante, le mélange réactionnel est purifié par chromatographie sur colonne de silice (éluant : CH₂Cl₂/MeOH : 95/5) pour conduire au produit attendu sous la forme d'une poudre jaune.
*Point de fusion :* 137-139 °C.
*IR*(KBr, cm⁻¹): 2865 (νCH de OCH₃), 1740 (νC=O), 1655 (νC=O), 1590-1575 (νC=C).
*SM* (Electrospray, m/z) : 413,4 (M⁺).

### Stade E : 3-[3-(2-Hydroxy-4-méthoxyphényl)-1,3-dioxoprop-1-yl]-1H-1-phényl-1,4-dihydroquinoléin-4-one :

Sous atmosphère inerte et à température ambiante, 12 mmoles de *tert*-butylate de potassium sont ajoutées lentement à 10 mmoles du composé obtenu au stade précédent en solution dans un mélange de diméthylformamide et de tétrahydrofurane (35/75). Le mélange réactionnel est agité pendant 2 h, puis versé sur une solution de 55 mL d'eau à 0°C contenant 1,3 mL d'acide chlorhydrique à 10 %. Le précipité obtenu est filtré, rincé abondamment à l'eau, puis séché. Le solide obtenu est purifié par chromatographie sur colonne de silice (éluant : CH₂Cl₂) pour conduire au produit attendu sous la forme d'un mélange céto-énolique.
*Point de fusion* : 228-230 °C.

### Stade F: 3-(7-Méthoxy-4-oxo-4H-1-benzopyran-2-yl)-1-phényl-1H-1,4-dihydroquinoléin-4-one :

A 10 mmoles du composé obtenu au stade précédent en solution dans 25 mL d'acide acétique glacial sont ajoutés lentement 25 mL d'une solution d'acide acétique contenant 20% d'acide sulfurique. Il se forme un précipité jaune. Après 2h30 à température ambiante, le mélange est versé sur de l'eau glacée (4°C). L'insoluble est filtré et rincé abondamment à l'eau pour conduire au produit attendu sous la forme d'une poudre blanche.
*Point de fusion* : 297 °C.
*IR* (cm⁻¹) : 2825 (νOCH₃), 1750 (νC=O), 1675 (νC=O), 1570-1590 (νC=C).
*SM* (Electrospray, m/z) : 396,1 (M⁺).

| ***Microanalyse élémentaire :*** | | | |
|---|---|---|---|
| | *% C* | *%H* | *%N* |
| ***Calculé :*** | *72,63* | *4,63* | *3,39* |
| ***Trouvé*** | *72,46* | *4,57* | *3,27* |

### EXEMPLE 2 : 3-(7-Hydroxy-4-oxo-4H-1-benzopyran-2-yl)-1-phényl-1H-quinoléin-4-one :

A 500 mmoles de phénol en solution dans 214 mL d'acide iodhydrique (solution aqueuse à 57 %) sont ajoutées, sous agitation, sous atmosphère inerte, et à l'abri de la lumière, 10 mmoles du composé de l'exemple 1. Le mélange réactionnel hétérogène est ensuite porté à 160°C pendant 15h. La solution, initialement jaune, vire à l'orange. Après retour à température ambiante, la solution est versée sur de la glace, et le précipité obtenu est rincé avec de l'eau, puis avec de l'éther diéthylique afin d'éliminer le phénol résiduel, pour conduire après recristallisation au produit attendu sous la forme d'une poudre jaune.
*Point de fusion*: 295-300 °C (acétone)
*IR* (cm⁻¹) 3280 (νOH), 1770 (νC=O), 1655 (νC=O), 1570-1590 (νC=C).
*SM* (Electrospray, m/z) : 381,1 (M⁺).

| ***Microanalyse élémentaire :*** | | | |
|---|---|---|---|
| | %C | *%H* | *%N* |
| *Calculé :* | *72, 17* | *4,29* | *3,51* |
| *Trouvé :* | *72,37* | *4,37* | *3,55* |

### EXEMPLE 3 : 3-(6-Méthoxy-4-oxo-4H-1-benzopyran-2-yl)-1-phényl-1H-1,4-dihydroquinoléin-4-one :

Le produit attendu est obtenu selon le procédé décrit dans l'exemple 1 en remplaçant, dans le stade D, la 2-hydroxy-4-méthoxyacétophénone par la 2-hydroxy-5-méthoxyacétophénone.
*Point de fusion* : 265°C.
*IR* (cm⁻¹) : 2830 (νOCH₃), 1740 (νC=O), 1655 (νC=O), 1570-1590 (νC=C).
*SM* (Electrospray, m/z) : 396,1 (M⁺).

| ***Microanalyse élémétaire:*** | | | |
|---|---|---|---|
| | %*C* | *%H* | *%N* |
| *Calculé :* | *72,63* | *4,63* | *3,39* |
| *Trouvé* : | *72,74* | *4,46* | *3,36* |

### EXEMPLE 4: 3-(5-Méthoxy-4-oxo-4H-1-benzopyram-2-yl)-1-phenyl-1H-1,4-dihydroquinoléin-4-Óne:

Le produit attendu est obtenu selon le procédé décrit dans l'exemple 1 en remplaçant, dans le stade D, la 2-hydroxy-4-méthoxyacétophénone par la 2-hydroxy-6-méthoxyacétophénone.
*Point de fusion:* 271 °C.
*IR* (cm⁻¹): 2830 (νOCH₃), 1744 (νC=O), 1655 (νC=O), 1570-1590 (νC=C),
*SM* (Electrospray, m/z): 396,1 (M⁺).

| ***Microanalyse élémentaire :*** | | | |
|---|---|---|---|
| | % C | *%H* | *%N* |
| *Calculé :* | *72,63* | *4,63* | *3,39* |
| *Trouvé :* | *72,46* | *4,57* | *3,27* |

### EXEMPLE 5: 3-(5,7-Diméthoxy-4-oxo-4H-1-benzopyran-2-yl)-1-phényl-1H-1,4-dihydroquinoléin-4-one :

### Stade A: 2,4-Diméthoxy-6-hydroxyacétophénone :

A 10 mmoles de phloroacétophénone monohydratée en solution dans l'acétone sont ajoutées, en une seule fois, 14,5 mmoles de K₂CO₃. Sous atmosphère inerte, 20 mmoles de sulfate de diméthyle sont ensuite additionnées sur une période de 30 min et le mélange réactionnel est porté au reflux de l'acétone pendant 12 h. Après retour à température ambiante, le mélange est versé sur de l'eau pour conduire à une suspension blanche qui est alors filtrée. La poudre blanche obtenue est lavée, puis recristallisée dans du méthanol pour conduire au produit attendu.
*Point de fusion :* 80-81 °C (méthanol).

### Stade B: 3-(5,7-Diméthoxy-4-oxo-4H-1-benzopyran-2-yl)-1-phényl-1H-1,4-dihydroquinoléin-4-one

Le produit attendu est obtenu selon le procédé décrit dans l'exemple 1 en remplaçant, dans le stade D, la 2-hydroxy-4-méthoxyacétophénone par le composé obtenu au stade A précédent.
*Point de fusion*: 282°C.
*IR* (cm⁻¹): 2825 (νOCH₃), 1744 (νC=O), 1655 (νC=O), 1570-1590 (νC=C).
SM (Electrospray, m/z) : 425,45 (M⁺).

| ***Microanalyse élémentaire :*** | | | |
|---|---|---|---|
| | %C | *%H* | *%N* |
| *Calculé :* | *70,42* | *4,77* | *3,16* |
| *Trouvé:* | *70,33* | *4,76* | *3,26* |

### EXEMPLE 6 : 3-(5-Hydroxy-4-oxo-4H-1-benzopyrana-2-yl)-1-phényl-1H-quinoléin-4-one :

Le produit attendu est obtenu selon le procédé décrit dans l'exemple 2 à partir du composé de l'exemple 4.
*Point de fusion:* >300 °C (acétone)
*IR* (cm⁻¹): 3200 (νOH), 1770 (νC=O), 1635 (νC=O), 1570-1590 (νC=C).
*SM* (Electrospray, m/z) : 381,1 (M⁺).

| ***Microanalyse élémentaire :*** | | | |
|---|---|---|---|
| | % C | *%H* | *%N* |
| *Calculé :* | *72,17* | *4, 29* | *3,51* |
| *Trouvé :* | *72,33* | *4,40* | *3,65* |

### EXEMPLE 7 : 3-(5-Hydroxy-7-méthoxy-4-oxo-4H-1-benzopyran-2-yl)-1-phenyl-1,H-quinoléin-4-one :

A 10 mmoles du composé de l'exemple 5 en suspension dans le dichlorométhane sont ajoutées pendant 15 min, sous atmosphère inerte et à l'abri de la lumière, 11 mmoles de BBr₃ (en solution 1M dans le dichlorométhane), entraînant la formation d'un précipité jaune. Le mélange réactionnel est fortement agité à température ambiante pendant 6 heures, puis refroidi à 0°C. De l'éthanol est alors ajouté et la solution est concentrée sous vide. Le résidu obtenu est ensuite versé sur une solution hydro-alcoolique (50 %), puis le milieu est vigoureusement agité pendant 10 minutes. Le précipité obtenu est filtré et rincé avec de l'eau, puis avec de l'éther diéthylique, pour conduire après recristallisation au produit attendu sous la forme d'une poudre beige.
*Point de fusion* : 295-296°C (acétone)
*IR* (cm⁻¹) : 3224 (νOH), 1780 (νC=O), 1655 (νC=O), 1570-1590 (νC=C).
*SM* (Electrospray, m/z) : 411,41 (M⁺).

| ***Microanalyse élémentaire :*** | | | |
|---|---|---|---|
| | %C | *%H* | *%N* |
| *Calculé :* | *72,99* | *4,16* | *3,40* |
| *Trouvé :* | *72,70* | *4,10* | *3,45* |

### EXEMPLE 8 : 3-(4-Oxo-4H-1-benzopyran-2-yl)-1-phényl-1H-4-one :

Le produit attendu est obtenu selon le procédé décrit dans l'exemple 1 en remplaçant dans le stade D, la 2-hydroxy-4-méthoxyacétophénone par l'acétophénone.
*Point de fusion* : 327-328°C.
*SM* (Electrospray, m/z) : 365,4 (M⁺).

| *Microanalyse élémentaire :* | | | |
|---|---|---|---|
| | %C | *%H* | *%N* |
| *Calculé:* | *78,89* | *4,14* | *3,83* |
| *Trouvé :* | *78,60* | *4,10* | *3,60* |

### EXEMPLE 9 : 3-[5,7-Diméthoxy-8-(1-méthyl-1,2,5,6-tétrahydropyridin-4-yl)-4-oxo-4H-1-benzopyran-2-yl]-1-phényl-1H-1,4-dihydroquinoléin-4-one:

### Stade A: 4-(2,4 Diméthoxy-6-hydroxy-5-méthylcarbonylphényl)-1-méthyl-1,2,5,6-tétrahydropyridine:

A 10 mmoles du composé obtenu au stade A de l'exemple 5 en solution dans l'acide acétique glacial, sont ajoutées lentement, de manière à ne pas dépasser 25 °C, 11,5 mmoles de 1-méthylpipéridin-4-one. Lorsque l'addition est terminée, on fait barboter un courant d'acide chlorhydrique gazeux pendant 1 h 40, puis le mélange réactionnel est chauffé à une température comprise entre 95 et 100°C pendant 5 h. L'acide acétique est éliminé par distillation sous vide, puis l'huile résiduelle reprise de l'eau et extraite à l'éther diéthylique. La phase aqueuse est rendue basique par addition d'une solution de NaOH à 40 %. Le précipité obtenu est filtré, rincé abondamment à l'eau et recristallisé dans l'éther de pétrole pour conduire au produit attendu.
*Point de fusion:* 143-144°C_{:}
*IR* (KBr, cm⁻¹): 3400-3200 (νOH), 2843 (νOCH₃), 1680 (νC=O), 1655 (νC=C).
*SM* (IE, m/z): 291 (M⁺).

### Stade B : 3-[5,7-Diméthoxy-8-(1-méthyl-1,2,5,6-tétrahydropyridinyl)-4-oxo-4H-1-benzopyran-2-yl]-1-phényl-1H-1,4-dihydroquinoléin-4-one :

Le produit attendu est obtenu selon le procédé décrit dans l'exemple 1 en remplaçant, dans le stade D, la 2-hydroxy-4-méthoxyacétophénone par le composé obtenu au stade A précédent.
*Point de fusion:* 248-250°C (acétone).
*IR* (cm⁻¹): 2835 (νOCH₃), 1755 (νC≡O), 1675 (νC=O), 1570-1590 (νC=C).
*SM* (Electrospray, m/z): 520,6 (M⁺).

| *Microanalyse élémentaire* : | | | |
|---|---|---|---|
| | *%C* | *%H* | *%N* |
| *Calculé :* | *73,28* | *5,10* | *5,22* |
| *Trouvé :* | *73,83* | *5,42* | *5,38* |

### EXEMPLE 10 : 3-(5,7-Dihydroxy-4-oxo-4H-1-benzopyran 2-yl)-1-phényl-1H-1,4-dihydroquinoléin-4-one :

Le produit attendu est obtenu selon le procédé décrit dans l'exemple 2 à partir du composé de l'exemple 5.
*Point de fusion :* 365-368°C (acétone).
*IR* (cm⁻¹) : 3224 (νOH), 1780 (νC=O), 1655 (νC=O), 1570-1590 (νC=C).
*SM* (Electrospray, m/z) : 397,4 (M⁺).

| ***Microanalyse élémentaire:*** | | | |
|---|---|---|---|
| | %C | *%H* | *%N* |
| *Calculé :* | *12,54* | *3,80* | *3,52* |
| *Trouvé :* | *72,20* | *4, 01* | *3,33* |

### EXEMPLE 11 : [2-(4-Oxo-1-phényl-1H-1,4-dihydroquinoléin-3-yl)-4H-1-benzopyran-7-yloxy]acétate d'éthyle :

A 10 mmoles du composé de l'exemple 2 en suspension dans l'acétone sont ajoutées lentement 20 mmoles de carbonate de potassium, puis 20 mmoles de bromoacétate d'éthyle. Le mélange est porté au reflux du solvant pendant 2 h 30, puis, après retour à température ambiante, versé sur de l'eau. L'insoluble obtenu est filtré et rincé abondamment à l'eau pour conduire au produit attendu sous la forme d'un solide blanc.
*Point de fusion* : 330°C.
*IR* (cm⁻¹) : 1744 (νC=O), 1680 (νC=O), 1655 (νC=O), 1570-1590 (νC=C).
*SM* (Electrospray, m/z) : 467,48 (M⁺).

| *Microanalyse élémentaire* : | | | |
|---|---|---|---|
| | %C | *%H* | *%N* |
| *Calculé :* | *71,94* | *4,53* | *3,00* |
| *Trouvé* | *71,70* | *4,77* | *3,40* |

### EXEMPLE 12 : 3-[5,7-Diméthoxy-(1-méthyl-1,2,5,6-tétrahydropyridin-4-yl)-4-oxo-4H-1-benzopyran-2-yl]-1-méthyl-1H-1,4-dihydroquinoléin-4-one :

### Stade A : N-phénylaminométhylènemalonate de diéthyle :

Le produit attendu est obtenu selon le procédé décrit dans le stade A de l'exemple 1, en remplaçant la diphénylamine par l'aniline.
*Point de fusion :* 46-48°C (hexane).
*SM(IE, m*/*z) :* 263 (M⁺).

### Stade B : N-Méthyl-N-phénylaminométhylènemalonate de diéthyle :

A 10 mmoles du composé obtenu au stade précédent en solution dans le tétrahydrofurane sont ajoutées, lentement (par petites portions) et sous atmosphère inerte, 12 mmoles de NaH à 95 %, puis, au goutte à goutte, 30 mmoles d'iodométhane. Le mélange réactionnel est ensuite maintenu sous agitation, à température ambiante et sous atmosphère inerte, pendant 12 h. 1 mL de méthanol est ajouté pour neutraliser l'excès d'hydrure de sodium. La solution est ensuite concentrée sous vide, puis de l'eau est ajoutée à l'huile résiduelle obtenue. Après extraction par le dichlorométhane, les phases organiques rassemblées sont séchées, filtrées et concentrées sous pression réduite pour conduire au produit attendu sous la forme d'une huile incolore.
*SM* (IE, m/z) : 276 (M⁺).

### Stade C : 3-[5,7-Diméthoxy-8-(1-méthyl-1,2,5,6-tétrahydropyridin-4-yl)-4-oxo-4H-1-benzopyran-2-yl]-1-méthyl-1H=1,4-dihydroquinoléin-4-one :

Le produit attendu est obtenu selon le procédé décrit dans les stades B à F de l'exemple 1 en remplaçant, au stade B, le composé obtenu au stade A de l'exemple 1 par le composé obtenu au stade B précédent, et en remplaçant, au stade D, la 2-hydroxy-4-méthoxyacétophénone par le composé obtenu au stade A de l'exemple 9.
*Point de fusion* : 289-291°C (acétone).
*IR* (cm⁻¹) : 2835 (νOCH₃), 1755 (νC=O), 1675 (νC=O), 1570-1590 (νC=C).
*SM* (Electrospray, m/z): 458,5 (M⁺).

| *Microanalyse élémentaire*: | | | |
|---|---|---|---|
| | %C | *%H* | *%N* |
| *Calculé:* | *70,73* | *5,72* | *6,11* |
| *Trouvé :* | *70, 25* | *5,48* | *5,78* |

### EXEMPLE 13 : 3-[5,7-Diméthoxy-8-[1-(4-fluorobenzyl)-1,2,5,6-tétrahydropyridin-4-yl]-4-oxo-4H-1-benzopyran-2-yl]-1-phényl-1H-1,4-dihydroquinoléin-4-one :

### Stade A: 1-(4-Fluorobenzyl)pipéridin-4-one :

A 10 mmoles de chlorhydrate de pipéridin-4-one monohydratée et 20 mmoles de triéthylamine en solution dans le dichlorométhane sont additionnées lentement 10 mmoles de chlorure de 4-fluorobenzyle, puis le mélange réactionnel est porté au reflux du solvant pendant 48 h sous forte agitation. Après retour à température ambiante, de l'eau est ajoutée, puis, une fois décantée, la phase organique est séchée, filtrée et concentrée sous vide pour conduire au produit attendu sous la forme d'une huile orange.
*SM* (IE, m/z) : 207,2 (M⁺).

### Stade B: 4-(3-Acétyl-4,6-diméthoxy-2-hydroxyphényl]-1-(4-fluorobenzyl)-1,2,5,6-tétrahydropyridine :

A 10 mmoles du composé obtenu au stade A de l'exemple 5 en solution dans l'acide acétique glacial sont ajoutées lentement, de manière à ne pas dépasser 25 °C, 11 mmoles du composé obtenu au stade A précédent. Lorsque l'addition est terminée, un courant d'acide chlorhydrique traverse la solution pendant 2 h, puis le mélange réactionnel est chauffé à une température comprise entre 95 et 100 °C pendant 5 h. L'acide acétique est éliminé par distillation sous vide, puis de l'eau est ajoutée à l'huile résiduelle obtenue. Après extraction par l'éther diéthylique, la phase aqueuse est rendue basique par une solution de NaOH à 40 %. Le précipité obtenu est filtré, rincé abondamment à l'eau et recristallisé dans un mélange éther / acétate d'éthyle (90/10) pour conduire au produit attendu sous la forme d'un solide beige.
*Point de fusion :* 147-150°C.
IR (KBr, cm⁻¹) : 3400-3200 (νOH), 2871 (νCH de OCH₃), 1633 (νC=O), 1655 (νC=C), 1350-1100 (νC-F).
*SM* (IE, m/z) : 385,4 (M⁺).

### Stade C: 3-[5,7-Diméthoxy-8-[1-(4-fluorobenzyl)-1,2,5,6-tétrahydropyridin-4 yl]-4-oxo-4H-1-benzopyran-2-yl]-1-phényl-1H-1,4-dihydroquinoléin-4-one :

Le produit attendu est obtenu selon le procédé décrit dans l'exemple 1 en remplaçant, au stade D, la 2-hydroxy-4-méthoxyacétophénone par le composé obtenu au stade B précédent.
*Point de fusion* : 218-219°C (acétone).
*IR* (cm⁻¹) : 2835 (νOCH₃), 1755 (νC=O), 1655 (νC=O), 1570-1590 (νC=C).
*SM* (Electrospray, m/z) : 614,66 (M⁺).

### EXEMPLE 14 : 3-[5,7-Dihydroxy-8-(1-méthyl-1,2,5,6-tétrahydropyridin-4-yl)-4-oxo-4H-1-benzopyran-2-yl]-1-phényl-1H-1,4-dihydroquinoléin-4-one :

10 mmoles du composé de l'exemple 9 sont dispersées dans du chlorure de pyridinium, puis le mélange est porté à 180°C, en tube scellé, pendant 12 heures. Refroidi à 100°C, le mélange réactionnel est ensuite versé sur de l'eau et le pH est ajusté à 7-8 à l'aide d'une solution d'hydrogénocarbonate de sodium à 10 % (le pH est initialement de 1). L'insoluble est séparé par filtration et rincé par de l'eau pour conduire au produit attendu.
*Point de fusion* : >250°C.
*IR* (cm⁻¹) : 3330 (νOH), 1785 (νC=O), 1665 (νC=O), 1570-1590 (νC=C).
*SM* (Electrospray, m/z) : 492,5 (M⁺).

| *Microanalyse élémentaire :* | | | |
|---|---|---|---|
| | *%C* | *%H* | *%N* |
| ***Calculé :*** | *72,94* | *5,12* | *5,87* |
| ***Trouvé :*** | *73,16* | *4,91* | *5,69* |

### EXEMPLE 15 : 3-[5,7-Diméthoxy-8-(1-benzyl-1,2,5,6-tétrahydropyridin-4-yl)-4-oxo-4H-1-benzopyran-2-yl]-1-phényl-1H-1,4-dihydroquinoléin-4-one ;

Le produit attendu est obtenu selon le procédé décrit dans les exemples B à C de l'exemple 13, en remplaçant, au stade B, le composé obtenu au stade A de l'exemple 13 par la 1-benzylpipéridin-4-one.
*Point de fusion* : 248-249°C (acétone).
*IR* (cm⁻¹): 2830 (νCH de OCH₃), 1765 (νC=O), 1655 (νC=O), 1570-1590 (νC=C).
*SM* (Electrospray, m/z) : 596,9 (M⁺).

| *Microanalyse élémentaire:* | | | |
|---|---|---|---|
| | %C | *%H* | *%N* |
| *Calculé:* | *76,49* | *5,41* | *4,69* |
| *Trouvé:* | *76, 06* | *5,03* | *4,93* |

### EXEMPLE 16: 3-[5,7-Dibydroxy-8-(1-benzyl-1,2,5,6-tétrahydropyridin-4-yl)-4-oxo-4H-1-benzopyran-2-yl]-1-phényl-1H-1,4-dihydroquinoléin-4-one:

Le produit attendu est obtenu selon le procédé décrit dans l'exemple 14 à partir du composé de l'exemple 15.
*Point de fusion* : 230-231°C (acétone).
*IR* (cm⁻¹): 3340 (νOH), 1752 (νC=O), 1655 (νC=O), 1570-1590 (νC=C).
*SM* (Electrospray, m/z) : 568,6 (M⁺).

| *Microanalyse élémentaire:* | | | |
|---|---|---|---|
| | %C | *%H* | *%N* |
| *Calculé:* | *76,04* | *4,96* | *4,93* |
| *Trouvé:* | *76,39* | *5,41* | *5,22* |

### EXEMPLE 17: 3-[5,7-Dihydroxy-8-[1-(4-fluorobenzyl)-1,2,5,6-tetrahydropyridin-4-yl]-4-oxo-4H-1-benzopyran-2-yl]-1-phényl-1H-1,4-dihydroquinoléin-4-one :

Le produit attendu est obtenu selon le procédé décrit dans l'exemple 14 à partir du composé de l'exemple 13.
*Point de fusion:* 197-198°C (acétone).
*IR* (cm⁻¹) : 3245 (νOH), 1775 (νC=O), 1655 (νC=O), 1570-1590 (νC=C).
*SM* (Electrospray, m/z) : 586,6 (M⁺).

| *Microanalyse élémentaire:* | | | |
|---|---|---|---|
| | %C | *%H* | *%N* |
| *Calculé :* | *73, 71* | *4, 64* | *4,78* |
| *Trouvé :* | *73, 20* | *4, 28* | *4,34* |

### EXEMPLE 18 : 3-[5,7-Diméthoxy-8-[1-(4-méthoxyhenzyl)-1,2,5,6-tétrahydropyridin-4-yl]-4-oxo-4H-1-benzopyran-2-yl]-1-phényl-1H-1,4-dihydroquinoléin-4-one :

Le produit attendu est obtenu selon le procédé décrit dans l'exemple 13 en remplaçant, au stade A, le chlorure de 4-fluorobenzyle par le chlorure de 4-méthoxybenzyle.
*Point de fusion* : 232-235°C (acétone).
*IR* (cm⁻¹) : 2845 (νOCH₃), 1750 (νC=O), 1655 (νC=O), 1570-1590 (νC=C).
*SM* (Electrospray, m/z) : 626,7 (M⁺):

### EXEMPLE 19: 3-[5,7-Diméthoxy-8-(1,2,5,6-tétrahydropyridin-4-yl)-4-oxo-4H-1-benzopyran-2-yl]-1-phényl-1H-1,4-dihydroquinoléin-4-one :

A 10 mmoles du composé de l'exemple 18 en suspension dans l'acide acétique glacial sont ajoutés, sous atmosphère inerte, 0,9 mg (10 % en masse) de Pd/C. Le mélange réactionnel est porté à 70°C et agité sous atmosphère d'hydrogène à la pression atmosphérique pendant 5 h. Le milieu réactionnel est ensuite filtré sur célite, puis lavé avec du méthanol. Les solvants sont éliminés par distillation sous vidé. Au résidu obtenu est ajoutée de l'eau dont le pH est ajusté à 8-9. L'extraction par le mélange CH₂Cl₂ / MeOH (90/10) conduit au produit attendu sous la forme d'un solide blanc.
*Point de fusion* : 251-253°C (éther diéthylique / acétone).
*IR* (cm⁻¹) : 3387 (νN-H), 2880 (νOCH₃), 1780 (νC=O), 1655 (νC=O), 1570-1590 (νC=C). SM (Electrospray, m/z) : 506,5 (M⁺).

| *Microanalyse élémentaire:* | | | |
|---|---|---|---|
| | %C | *%H* | *%N* |
| *Calculé* : | *73,50* | *5,17* | *5,53* |
| *Trouvé :* | *73,12* | *5,58* | *4,98* |

### EXEMPLE 20: 3-[5,7-Diméthoxy-8-(1-isopropyl-1,2,5,6-tétrahydropyridinyl)-4-oxo-4H-1-benzopyran-2-yl]-1-phényl-1H-1,4-dihydroquinoléin-4-one :

Le produit attendu est obtenu selon le procédé décrit dans les exemples B à C de l'exemple 13, en remplaçant, au stade B, le composé obtenu au stade A de l'exemple 13 par la N-isopropylpipéridin-4-one.
*Point de fusion* : 248-249°C (acétone).
*IR* (cm⁻¹) : 2850 (νOCH₃),1755 (νC=O), 1650 (νC=O), 1570-1590 (νC=C).
*SM* (Electrospray, m/z) : 548,6 (M⁺).

| *Microanalyse élémentaire :* | | | |
|---|---|---|---|
| | %C | *%H* | *%N* |
| *Calculé:* | *74,43* | *5,88* | *5,11* |
| *Trouvé :* | *73, 78* | *5,23* | *5,78* |

### EXEMPLE 21 : 3-[7-(4-Bromobenzyloxy)-4-oxo-4H-1-benzopyran-2-yl]-1-phényl-1H-quinoléin-4-one :

Le produit attendu est obtenu selon le procédé décrit dans l'exemple 11 à partir du composé de l'exemple 2, en remplaçant le brômoacétate d'éthyle par le chlorure de 4-bromobenzyle.
*IR* (cm⁻¹) : 1744 (νC=O), 1655 (νC=O), 1570-1590 (νC=C).
*SM* (Electrospray, m/z): 549,4 (M⁺).

| *Microanalyse élémentaire:* | | | |
|---|---|---|---|
| | *% C* | *%H* | *%N* |
| *Calculé :* | *67,65* | *3,66* | *2,54* |
| *Trouvé :* | *66,95* | *4,21* | *2,44* |

### EXEMPLE 22 : 3-[5,7-Diméthoxy-8-[1-(2-diméthylaminoéthyl)-1,2,5,6-tétrahydropyridin-4-yl]-4-oxo-4H-1-benzopyran-2-yl]-1-phényl-1H-1,4-dihydroquinoléin-4-one :

Le produit attendu est obtenu selon le procédé décrit dans l'exemple 13, en remplaçant, au stade A, le chlorure de 4-fluorobenzyle par la 2-chloro-N,N-diméthyléthylamine.

### EXEMPLE 23 : 3-[5,7-Dihydroxy-8-[1-(2-diméthylaminoéthyl)-1,2,5,6-tétrahydropyridin-4-yl]-4-oxo-4H-1-benzopyran-2-yl]-1-phényl-1H-1,4-dihydroquinoléin-4-one :

Le produit attendu est obtenu selon le procédé décrit dans l'exemple 2, à partir du composé de l'exemple 22.

### ETUDE PHARMACOLOGIOUE DES COMPOSES DE L'INVENTION

### EXEMPLE 24 : Étude in vitro de la cytotoxicité propre des dérivés de l'invention

Onze lignées cellulaires provenant de cancers de différentes origines et localisations (poumon, sein, prostate, colon, sang, vessie, peau, ovaire, cerveau) sont maintenues en culture pour étudier les différents composés, en comparaison à la substance de référence.
Ces cellules sont incubées pendant 96 heures avec différentes concentrations des dérivés de l'invention.

L'activité cytotoxique *in vitro* est déterminée par le test au MTT [bromure de 3-(4,5-dimethylthiazol-2-yl)-2,5-diphényltétrazolium] tel que décrit par Carmichael dans Cancer Res. - 1987 ; 47 (4) : 936-942.
Cette activité est exprimée en IC₅₀, c'est-à-dire en concentration qui inhibe de 50 % la prolifération des cellules tumorales.

Dans ce modèle, les dérivés de l'invention possèdent une activité cytotoxique propre, sur une ou plusieurs lignées de cellules tumorales.
A titre d'exemple, le composé de l'exemple 9 présente une activité cytotoxique sur 8 des 11 lignées testées (IC₅₀ comprise entre 2 et 10 µM suivant la lignée cellulaire utilisée).

Le composé de l'exemple 14 présente, lui, une IC₅₀ comprise entre 0,05 et 0,25 µM, sur 5 des 11 lignées testées.

### EXEMPLE 25 : Effet synergique des dérivés de l'invention en combinaison avec des anticancéreux connus in vitro

Trois lignées cellulaires sensibles à trois anticancéreux sont utilisées : des cellules de cancer du sein en association au traitement par le tamoxifène (TXL), des cellules de cancer du poumon en association au traitement par le cisplatine (CDDP), des cellules de cancer du colon en association au traitement par le SN38, métabolite du CPT-11 (irinotécan). Les cellules tumorales sont incubées pendant 96 heures avec cinq concentrations différentes de chacun des dérivés de l'invention et cinq concentrations de chacun des anticancéreux en association.
L'activité cytotoxique *in vitro* est déterminée le test au MTT cité à l'exemple 24.

L'analyse des données est réalisée selon la méthode de Chou et Talabay, publiée dans Trends Pharmaceutical Sci. - 1983 ; 4 : 450.

Les dérivés de l'invention montrent un effet synergique avec les différents anticancéreux testés, c'est-à-dire qu'ils renforcent l'activité cytotoxique de l'anticancéreux administré simultanément.

A titre d'exemple, les composés des exemples 14 et 16 montrent un effet synergique, aussi bien avec le paclitaxel qu'avec le cisplatine.

### EXEMPLE 26 : Effet apoptotique des dérivés de l'invention

L'apoptose est un mécanisme naturel qui permet à l'organisme humain de se débarrasser de cellules anormales comme les cellules cancéreuses.

L'étude des effets pro-apoptotiques des dérivés de l'invention est réalisée sur une lignée de cancer de la prostate (LN Cap). Les cellules ont été incubées pendant des durées variant de 8 à 96 heures à la concentration de l'IC₅₀.

Le test TUNEL était ensuite pratiqué selon la méthode décrite par Sgonc dans Trends Genetics - 1994 ; 10 : 41.

Les dérivés de l'invention sont capables d'induire une apoptose dont le maximum d'intensité se présente à des temps différents selon les dérivés.

Les dérivés de l'invention diffèrent des substances antérieurement décrites par leur capacité d'induire une apoptose plus précocement. A titre d'exemple, le composé de l'exemple 6 induit précocement (8 heures) une apoptose très importante.

### EXEMPLE 27 : Composition pharmaceutique

| | |
|---|---|
| Formule de préparation pour 1000 comprimés dosés à 10 mg | |
| Composé de l'exemple 9 | 10 g |
| Hydroxypropylcellulose | 2g |
| Amidon de blé | 10 g |
| Lactose | 100 g |
| Stéarate de magnésium | 3 g |
| Talc | 3 g |

## Revendications

1. Composé de formule (I) : 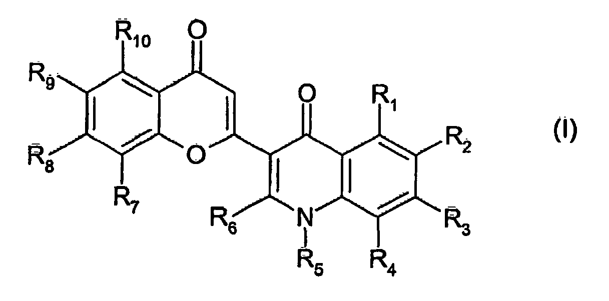 dans laquelle :
• R₁, R₂, R₃, R₄, R₆, R₈, R₉ et R₁₀, identiques ou différents, représentent chacun un groupement choisi parmi hydrogène, hydroxy, alkoxy (C₁-C₆) linéaire ou ramifié, alkyle (C₁-C₆) linéaire ou ramifié, arylalkoxy dans lequel le goupement alkoxy est (C₁-C₆) linéaire ou ramifié, alkoxycarbonylalkoxy dans lequel chacun des groupements alkoxy est (C₁-C₆) linéaire ou ramifié, et OR' dans lequel R' représenté un groupement ionisé ou ionisable,
• R₅ représente un groupement choisi parmi alkyle (C₁-C₆) linéaire ou ramifié, aryle et hétéroaryle,
• R₇ représenté un groupement choisi parmi hydrogène, hydroxy, alkoxy (C₁-C₆) linéaire ou ramifié, alkyle (C₁-C₆) linéaire ou ramifié et cycloalkyle (C₃-C₇), ou bien R₇ représente un hétérocycle azoté ou oxygéné,
ses isomères optiques lorsqu'ils existent, ses sels d'addition à un acide pharmaceutiquement acceptable ainsi que ses hydrates et ses solvates,
étant entendu que par groupement aryle, on entend phényle, biphénylyle, naphtyle, ou tétrahydronaphtyle, chacun de ces groupements étant éventuellement substitué par un ou plusieurs atomes ou groupements, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, amino (substitué éventuellement par un ou deux groupements alkyle (C₁-C₆) linéaire ou ramifié), nitro, ou alkylènedioxy (C₁-C₂),
par groupement hétéroaryle, on entend un groupement de 5 à 12 chaînons, soit monocyclique aromatique, soit bicyclique dont l'un au moins des cycles possède un caractère aromatique, et contenant un, deux ou trois hétéroatomes choisis parmi oxygène, azote ou soufre, étant entendu que l'hétéroaryle peut être éventuellement substitué par un ou plusieurs atomes ou groupements, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, ou amino (substitué éventuellement par un ou plusieurs groupements alkyle (C₁-C₆) linéaire ou ramifié),
par hétérocycle azoté, on entend un groupement monocyclique, saturé ou insaturé, de 5 à 7 chaînons, contenant un atome d'azote, et éventuellement substitué par un ou plusieurs groupements choisis parmi hydroxy, alkoxy (C₁-C₆) linéaire ou ramifié, alkyle (C₁-C₆) linéaire ou ramifié, arylalkyle (C₁-C₆) linéaire ou ramifié, aminoalkyle (C₁-C₆) linéaire ou ramifié dans lequel le groupement amino est éventuellement substitué par un ou deux groupements alkyle (C₁-C₆) linéaire ou ramifié,
et par hétérocyle oxygéné, on entend un groupement monocyclique, saturé ou insaturé, de 5 à 7 chaînons, contenant un atome d'oxygène, et éventuellement substitué par un ou plusieurs groupements choisis parmi hydroxy, alkoxy (C₁-C₆) linéaire ou ramifié, alkyle (C₁-C₆) linéaire ou ramifié, arylalkyle (C₁-C₆) linéaire ou ramifié, aminoalkyle (C₁-C₆) linéaire ou ramifié dans lequel le groupement amino est éventuellement substitué par un ou deux groupements alkyle (C₁-C₆) linéaire ou ramifié.

2. Composé de formule (I) selon la revendication 1 tel que R₁, R₂, R₃, R₄, R₆, R₈, R₉ et R₁₀, identiques ou différents, représentent chacun un groupement choisi parmi hydrogène, hydroxy, alkoxy (C₁-C₆) linéaire ou ramifié, alkyle (C₁-C₆) linéaire ou ramifié, arylalkoxy dans lequel le goupement alkoxy est (C₁-C₆) linéaire ou ramifié, et alkoxycarbonylalkoxy dans lequel chacun des groupements alkoxy est (C₁-C₆) linéaire ou ramifié.

3. Composé de formule (I) selon la revendication 1 tel que R₁, R₂, R₃, R₄, R₆, R₈, R₉ et R₁₀, identiques ou différents, représentent chacun un groupement choisi parmi hydrogène, hydroxy, alkoxy (C₁-C₆) linéaire ou ramifié, alkyle (C₁-C₆) linéaire ou ramifié, arylalkoxy dans lequel le goupement alkoxy est (C₁-C₆) linéaire ou ramifié alkoxycarbonylalkoxy dans lequel chacun des groupements alkoxy est (C₁-C₆) linéaire ou ramifié, et OR' dans lequel R' représente un groupement choisi parmi phosphate - PO(OH)₂, sulfate -SO₃H, carboxyalkylcarbonyle dans lequel le goupement alkyle est (C₁-C₆) linéaire ou ramifié, dialkylaminoalkylcarbonyle dans lequel chacun des goupements alkyle est (C₁-C₆) linéaire ou ramifié, et carboxyalkylaminocarbonylé, dans lequel le goupement alkyle est (C₁-C₆) linéaire ou ramifié.

4. Composé de formule (I) selon l'une quelconque des revendications 1 à 3 tel que R₅ représente un groupement aryle.

5. Composé de formule (I) selon l'une quelconque des revendications 1 à 4 tel que R₇ représente un atome d'hydrogène.

6. Composé de formule (I) selon l'une quelconque des revendications 1 à 4 tel que R₇ représente un hétérocycle azoté éventuellement substitué.

7. Composé de formule (I) selon l'une quelconque des revendications 1 à 6 tel que R₅ représente un groupement phényle et R₇ représente un atome d'hydrogène ou un groupement 1,2,3,6-tétrahydro-4-pyridyle substitué,

8. Composé de formule (I) selon la revendication 1 qui est la 3-(5-hydroxy-4-oxo-4*H*-1-benzopyran-2-yl)-1-phényl-1*H*-quinoléin-4-one.

9. Composé de formule (I) selon la revendication 1 qui est la 3-[5,7-diméthoxy-8=(1= méthyl-1,2,5,6-tétrahydropyridin-4-yl)-4-oxo-4*H*-1-benzopyran-2-yl]-1-phényl-1*H* 1,4-dihydroquinoléin-4-one.

10. Composé de formule (I) selon la revendication 1 qui est la 3-(5,7-dihydroxy-4-oxo-4*H*-1-benzopyran-2-yl)-1-phényl-1*H*-1,4-dihydroquinoléin-4-one.

11. Composé de formule (I) selon la revendication 1 qui est la 3-[5,7-dihydroxy-8-(1-méthyl-1,2,5,6-tétrahydropyridin-4-yl)-4-oxo-4*H*-1-benzopyran-2-yl]-1-phényl-1*H-*1,4-dihydroquinoléin-4-one.

12. Procédé de préparation des composés de formule (I) selon la revendication 1 **caractérisé en ce que** l'on met en réaction un composé de formule (II) : 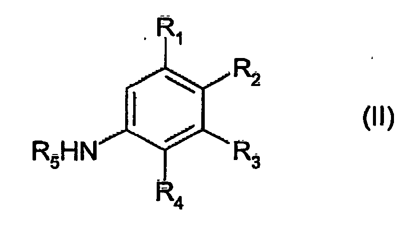 dans lequel R₁, R₂, R₃, R₄ et R₅ sont tels que définis dans la formule (I),
avec de l'éthoxyméthylène malonate de diéthyle, pour conduire au composé de formule (III): 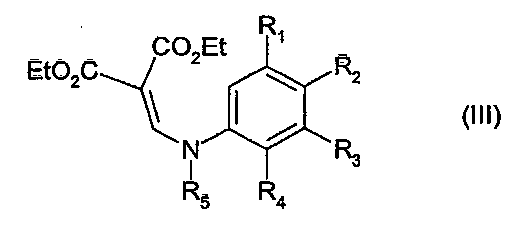 dans lequel R₁, R₂, R₃, R₄ et R₅ sont tels que définis précédemment, et Et représente le groupe éthyle,
que l'on cyclise dans des conditions acides, pour conduire au composé de formule (IV): 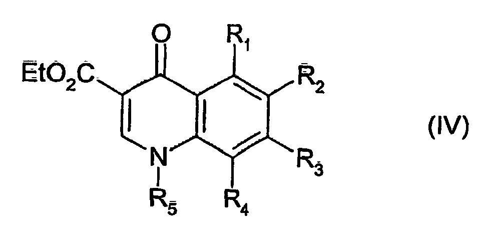 dans lequel R₁, R₂, R₃, R₄ et R₅ et Et sont tels que définis précédemment,
que l'on saponifie, pour conduire au composé de formule (V) : 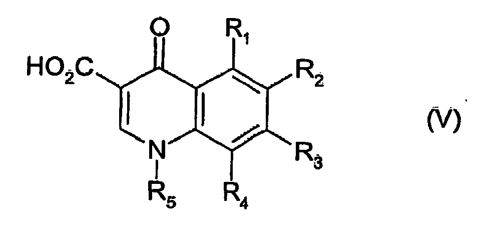 dans lequel R₁, R₂, R₃, R₄ et R₅ sont tels que définis précédemment,
que l'on transforme en chlorure d'acide par action de chlorure de thionyle, puis que l'on met en réaction avec le composé de formule (VI) : 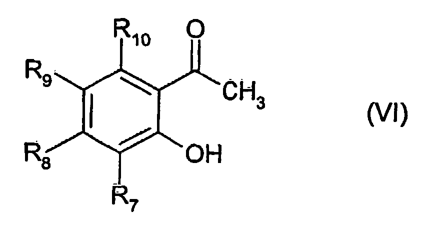 dans lequel R₇, R₈, R₉ et R₁₀ sont tels que définis dans la formule (I),
pour conduire au composé de formule (VII): 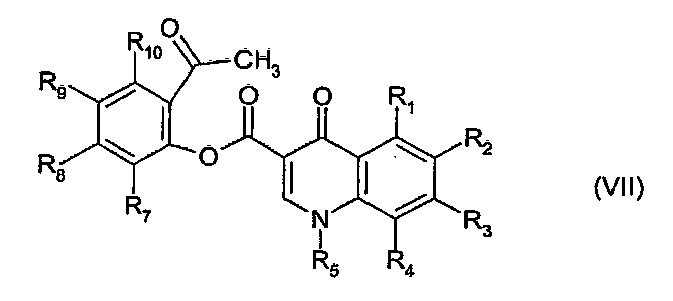 dans lequel R₁, R₂, R₃, R₄, R₅, R₆, R₇, R₈, R₉ et R₁₀ sont tels que définis précédemment,
que l'on soumet à l'action d'une base, pour conduire au composé de formule (VIII): 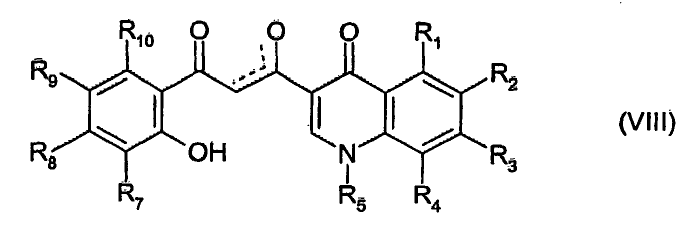 dans lequel R₁, R₂, R₃, R₄, R₅, R₆, R₇, R₈, R₉ et R₁₀ sont tels que définis précédemment, et 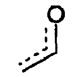 signifie que le composé est obtenu selon les molécules sous la forme d'un mélange céto-énolique,
que l'on soumet ensuite à des conditions acides pour conduire au composé de formule (I), que l'on purifie, le cas échéant, selon une technique classique de purification, dont on sépare, le cas échéant, les isomères optiques selon une technique classique de séparation, et que l'on transforme, si on le souhaite, en leurs sels d'addition à un acide pharmaceutiquement acceptable.

13. Composition pharmaceutique contenant comme principe actif un composé selon l'une quelconque des revendications 1 à 11, en combinaison avec un ou plusieurs véhicules inertes, non toxiques et pharmaceutiquement acceptables.

14. Composition pharmaceutique selon la revendication 13 utile en tant que médicament anticancéreux.

## Patentansprüche

1. Verbindung der Formel (I): 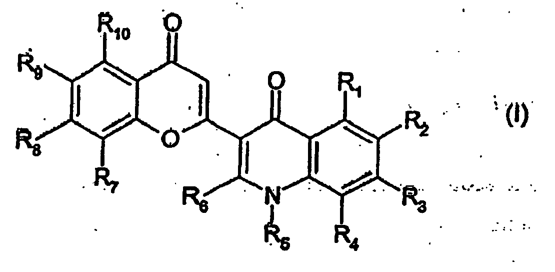 in der:
• R₁, R₂, R₃, R₄, R₆, R₈, R₉ und R₁₀, die gleichartig oder verschieden sind, jeweils eine Gruppe bedeuten ausgewählt aus Wasserstoff, Hydroxy, geradkettigem oder verzweigtem (C₁-C₆)-Alkoxy, geradkettigem oder verzweigtem (C₁-C₆)-Alkyl, Arylalkoxy, worin die Alkoxygruppe eine geradkettige oder verzweigte (C₁-C₆)-Alkoxygruppe ist, Alkoxycarbonylalkoxy, worin jede der Alkoxygruppen eine geradkettige oder verzweigte (C₁-C₆)-Alkoxygruppe ist, und OR', worin R' eine ionisierte oder ionisierbare Gruppe darstellt,
• R₅ eine Gruppe bedeutet ausgewählt aus geradkettigem oder verzweigtem (C₁₋C₆)-Alkyl, Aryl und Heteroaryl,
• R₇ eine Gruppe bedeutet ausgewählt aus Wasserstoff, Hydroxy, geradkettigem oder verzweigtem (C₁-C₆)-Alkoxy, geradkettigem oder verzweigtem (C₁-C₆)-Alkyl und (C₃-C₇)-Cycloalkyl, oder R₇ einen stickstoffhaltigen oder sauerstoffhaltigen Heterocyclus bedeutet,
ihre optischen Isomeren, falls sie existieren, und ihre Additionssalze mit einer pharmazeutisch annehmbaren Säure sowie ihre Hydrate und ihre Solvate, mit der Maßgabe, daß man unter einer Arylgruppe Phenyl, Biphenyl, Naphthyl oder Tetrahydronaphthyl versteht, wobei jede dieser Gruppen gegebenenfalls durch ein oder mehrere gleichartige oder verschiedenartige Atome oder Gruppen substituiert ist, ausgewählt aus Halogenatomen und geradkettigen oder verzweigten (C₁-C₆)-Alkylgruppen. Hydroxygruppen, geradkettigen oder verzweigten (C₁-C₆)-Alkoxygruppen, geradkettigen oder verzweigten (C₁-C₆)-Polyhalogenalkylgruppen, Aminogruppen (die gegebenenfalls durch eine oder zwei geradkettige oder verzweigte (C₁-C₆)-Alkylgruppen substituiert sind), Nitrogruppen oder (C₁-C₂)-Alkylendioxygruppen, man unter einer Heteroarylgruppe eine Gruppe mit 5 bis 12 Kettengliedern versteht, die entweder monocyclisch aromatisch ist oder bicyclisch, wobei mindestens einer der Ringe einen aromatischen Charakter besitzt, und die ein, zwei oder drei Heteroatome ausgewählt aus Sauerstoff, Stickstoff oder Schwefel enthält, mit der Maßgabe, daß die Heteroarylgruppe gegebenenfalls durch ein oder mehrere gleichartige oder verschiedene Atome oder Gruppen substituiert sein kann, ausgewählt aus Halogenatomen und geradkettigen oder verzweigten (C₁-C₆)-Alkylgruppen, Hydroxygruppen, geradkettigen oder verzweigten (C₁-C₆)-Alkoxygruppen, geradkettigen oder verzweigten (C₁-C₆)-Polyhalogenalkylgruppen oder Aminogruppen (die gegebenenfalls durch eine oder mehrere geradkettige oder verzweigte (C₁-C₆)-Alkylgruppen substituiert sind),
man unter einem stickstoffhaltigen Heterocyclus eine monocyclische, gesättigte oder ungesättigte Gruppe mit 5 bis 7 Kettengliedern versteht, die ein Stickstoffatom enthält und die gegebenenfalls durch eine oder mehrere Gruppen substituiert ist ausgewählt aus Hydroxy, geradkettigem oder verzweigtem (C₁-C₆)-Alkoxy, geradkettigem oder verzweigtem (C₁-C₆)-Alkyl, geradkettigem oder verzweigtem Aryl-(C₁₋C₆)-alkyl, geradkettigem oder verzweigtem (C₁-C₆)-Aminoalkyl, wobei die Aminogruppe gegebenenfalls durch eine oder zwei geradkettige oder verzweigte (C₁-C₆)-Alkylgruppen substituiert ist,
und man unter einem sauerstoffhaltigen Heterocyclus eine monocyclische, gesättigte oder ungesättigte Gruppe mit 5 bis 7 Kettengliedern versteht, die ein Sauerstoffatom enthält und gegebenenfalls durch eine oder mehrere Gruppen substituiert ist ausgewählt aus Hydroxy, geradkettigem oder verzweigtem (C₁-C₆)-Alkoxy, geradkettigem oder verzweigtem (C₁-C₆)-Alkyl, geradkettigem oder verzweigtem Aryl-(C₁₋C₆)-alkyl, geradkettigem oder verzweigtem (C₁-C₆)-Aminoalkyl, wobei die Aminogruppe gegebenenfalls durch eine oder zwei geradkettige oder verzweigte (C₁-C₆)-Alkylgruppen substituiert ist.

2. Verbindung der Formel (I) nach Anspruch 1, worin R₁, R₂, R₃, R₄, R₆, R₈, R₉ und R₁₀, die gleichartig oder verschieden sind, jeweils eine Gruppe bedeuten ausgewählt aus Wasserstoff, Hydroxy, geradkettigem oder verzweigtem (C₁-C₆)-Alkoxy, geradkettigem oder verzweigtem (C₁-C₆)-Alkyl, Arylalkoxy, worin die Alkoxygruppe eine geradkettige oder verzweigte (C₁-C₆)-Alkoxygruppe ist, und Alkoxycarbonylalkoxy, worin jede der Alkoxygruppen eine geradkettige oder verzweigte (C₁-C₆)-Alkoxygruppe darstellt.

3. Verbindung der Formel (I) nach Anspruch 1, worin R₁, R₂, R₃, R₄, R₆, R₈, R₉ und R₁₀, die gleichartig oder verschieden sind, jeweils eine Gruppe bedeuten ausgewählt aus Wasserstoff, Hydroxy, geradkettigem oder verzweigtem (C₁-C₆)-Alkoxy, geradkettigem oder verzweigtem (C₁-C₆)-Alkyl, Arylalkoxy, worin die Alkoxygruppe eine geradkettige oder verzweigte (C₁-C₆)-Alkoxygruppe ist, Alkoxycarbonylalkoxy, worin jede der Alkoxygruppen eine geradkettige oder verzweigte (C₁-C₆)-Alkoxygruppe ist, und OR', worin R' eine Gruppe bedeutet ausgewählt aus Phosphat -PO(OH)₂, Sulfat -SO₃H, Carboxyalkylcarbonyl, worin die Alkylgruppe eine geradkettige oder verzweigte (C₁-C₆)-Alkylgruppe ist. Dialkylaminoalkylcarbonyl, worin jede der Alkylgruppen eine geradkettige oder verzweigte (C₁-C₆)-Alkylgruppe ist, und Carboxyalkylaminocarbonyl, worin die Alkylgruppe eine geradkettige oder verzweigte (C₁₋C₆)-Alkylgruppe ist.

4. Verbindung der Formel (I) nach einem der Ansprüche 1 bis 3, worin R₅ eine Arylgruppe bedeutet.

5. Verbindung der Formel (I) nach einem der Ansprüche 1 bis 4, worin R₇ ein Wasserstoffatom bedeutet.

6. Verbindung der Formel (I) nach einem der Ansprüche 1 bis 4, worin R₇ einen gegebenenfalls substituierten stickstoffhaltigen Heterocyclus bedeutet.

7. Verbindung der Formel (I) nach einem der Ansprüche 1 bis 6, worin R₅ eine Phenylgruppe und R₇ ein Wasserstoffatom oder eine substituierte 1,2,3,6-Tetrahydro-4-pyridylgruppe bedeuten.

8. Verbindung der Formel (I) nach Anspruch 1, nämlich 3-(5-Hydroxy-4-oxo-4*H*-1-benzopyran-2-yl)-1-phenyl-1*H*-chinolin-4-on.

9. Verbindung der Formel (I) nach Anspruch 1, nämlich 3-[5,7-Dimethoxy-8-(1-methyl-1,2,5,6-tetrahydropyridin-4-y)-4-oxo-4*H*-1-benzopyran-2-yl]-1-phenyl-1*H*-1,4-dihydrochinolin-4-on.

10. Verbindung der Formel (I) nach Anspruch 1, nämlich 3-(5,7-Dihydroxy-4-oxo-4*H*-1-benzopyran-2-yl)-1-phenyl-1*H*-1,4-dihydrochinolin-4-on.

11. Verbindung der Formel (I) nach Anspruch 1, nämlich 3-[5,7-Dihydroxy-8-(1-methyl-1,2,5,6-tetrahydropyridin-4-yl)-4-oxo-4*H*-1-benzopyran-2-yl]-1-phenyl-1*H-*1,4-dihydrochinolin-4-on.

12. Verfahren zur Herstellung der Verbindungen der Formel (I) nach Anspruch 1, **dadurch gekennzeichnet, daß** man eine Verbindung der Formel (II): 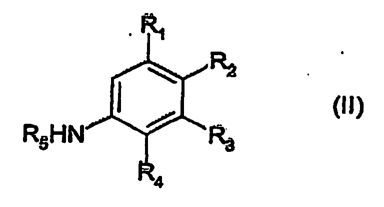 in der R₁, R₂, R₃, R₄ und R₅ die bezüglich der Formel (I) angegebenen Bedeutungen besitzen,
mit Ethoxymethylenmalonsäurediethylester umsetzt zur Bildung der Verbindung der Formel (III): 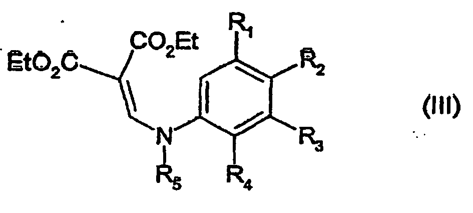 in der R₁, R₂, R₃, R₄ und R₅ die oben angegebenen Bedeutungen besitzen und Et die Ethylgruppe darstellt,
welche man unter sauren Bedingungen cyclisiert zur Bildung der Verbindung der Formel (IV): 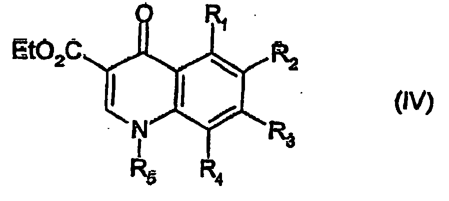 in der R₁, R₂, R₃, R₄, R₅ und Et die oben angegebenen Bedeutungen besitzen,
welche man verseift zur Bildung der Verbindung der Formel (V): 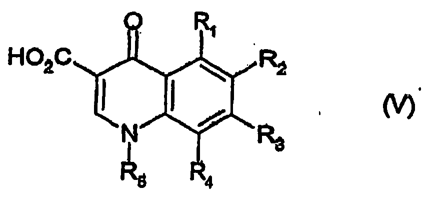 in der R₁, R₂, R₃, R₄ und R₅ die oben angegebenen Bedeutungen besitzen,
welche man durch Einwirkung von Thionylchlorid in das Säurechlorid umwandelt, welches man mit der Verbindung der Formel (VI) umsetzt: 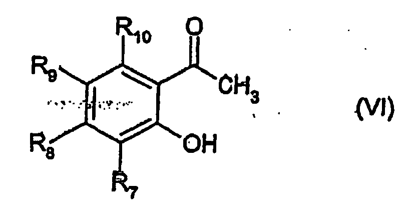 in der R₇, R₈, R₉ und R₁₀ die bezüglich der Formel (I) angegebenen Bedeutungen besitzen,
zur Bildung der Verbindung der Formel (VII): 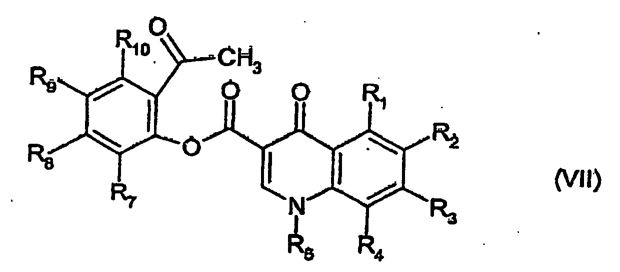 in der R₁, R₂, R₃, R₄, R₅, R₆, R₇, R₈, R₈ und R₁₀ die oben angegebenen Bedeutungen besitzen,
welche man der Einwirkung einer Base unterwirft zur Bildung der Verbindung der Formel (VIII): 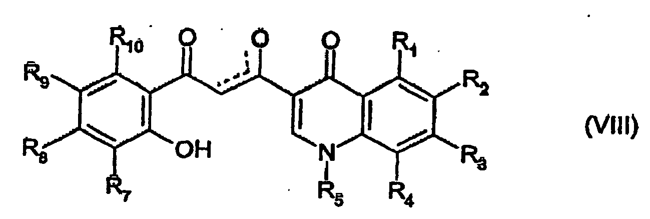 in der R₁, R₂, R₃, R₄, R₅, R₆, R₇, R₈, R₉ und R₁₀ die oben angegebenen Bedeutungen besitzen,
und das Symbol 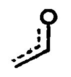 bedeutet, daß man die Verbindung in Abhängigkeit von den Molekülen in Form einer Keto-Enol-Mischung erhält,
welche man anschließend sauren Bedingungen unterwirft zur Bildung der Verbindung der Formel (I), welche man gegebenenfalls mit Hilfe einer klassischen Reinigungsmethode reinigt, welche man gegebenenfalls mit Hilfe einer klassischen Trennmethode in ihre optischen Isomeren trennt und welche man gewünschtenfalls in ihre Additionssalze mit einer pharmazeutisch annehmbaren Säure überführt.

13. Pharmazeutische Zubereitung enthaltend als Wirkstoff eine Verbindung nach einem der Ansprüche 1 bis 11 in Kombination mit einem oder mehreren inerten, nichttoxischen und pharmazeutisch annehmbaren Hilfsstoffen.

14. Pharmazeutische Zubereitung nach Anspruch 13 nützlich als Antikrebs-Arzneimittel.

## Claims

1. Compound of formula (I) : 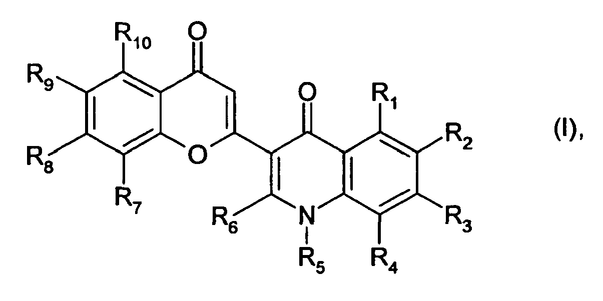 wherein :
• R₁, R₂, R₃, R₄, R₆, R₈, R₉ and R₁₀, which may be the same or different, each represent a group selected from hydrogen, hydroxy, linear or branched (C₁-C₆)alkoxy, linear or branched (C₁-C₆)alkyl, arylalkoxy in which the alkoxy group is linear or branched (C₁-C₆), alkoxycarbonylalkoxy in which each of the alkoxy groups is linear or branched (C₁-C₆), and OR' wherein R' represents an ionised or ionisable group,
• R₅ represents a group selected from linear or branched (C₁-C₆)alkyl, aryl and heteroaryl,
• R₇ represents a group selected from hydrogen, hydroxy, linear or branched (C₁-C₆)alkoxy, linear or branched (C₁-C₆)alkyl and (C₃-C₇)cycloalkyl, or R₇ represents a nitrogen-containing or oxygen-containing heterocycle,
optical isomers thereof when they exist, addition salts thereof with a pharmaceutically acceptable acid and hydrates and solvates thereof,
an aryl group being understood to be phenyl, biphenylyl, naphthyl or tetrahydronaphthyl, each of those groups being optionally substituted by one or more identical or different atoms or groups selected from halogen atoms and linear or branched (C₁-C₆)alkyl groups, hydroxy groups, linear or branched (C₁-C₆)alkoxy groups, linear or branched (C₁-C₆)polyhaloalkyl groups, amino groups (optionally substituted by one or two linear or branched (C₁-C₆)alkyl groups), nitro groups and (C₁-C₂)alkylenedioxy groups,
a heteroaryl group being understood to be a 5- to 12-membered group which either is monocyclic and aromatic or is bicyclic with at least one of the rings being of aromatic character, and which contains one, two or three hetero atoms selected from oxygen, nitrogen and sulphur, it being understood that the heteroaryl group may be optionally substituted by one or more identical or different atoms or groups selected from halogen atoms and linear or branched (C₁-C₆)alkyl groups, hydroxy groups, linear or branched (C₁-C₆)alkoxy groups, linear or branched (C₁-C₆)polyhaloalkyl groups and amino groups (optionally substituted by one or two linear or branched (C₁-C₆)alkyl groups),
a nitrogen-containing heterocycle being understood to mean a saturated or unsaturated, 5-to 7-membered monocyclic group containing a nitrogen atom and optionally substituted by one or more groups selected from hydroxy, linear or branched (C₁-C₆)alkoxy, linear or branched (C₁-C₆)alkyl, aryl-(C₁-C₆)alkyl in which the alkyl moiety is linear or branched and amino-(C₁-C₆)alkyl in which the alkyl moiety is linear or branched and in which the amino group is optionally substituted by one or two linear or branched (C₁-C₆)alkyl groups,
an oxygen-containing heterocycle being understood to mean a saturated or unsaturated, 5-to 7-membered monocyclic group containing an oxygen atom and optionally substituted by one or more groups selected from hydroxy, linear or branched (C₁-C₆)alkoxy, linear or branched (C₁-C₆)alkyl, aryl-(C₁-C₆)alkyl in which the alkyl moiety is linear or branched and amino-(C₁-C₆)alkyl in which the alkyl moiety is linear or branched and in which the amino group is optionally substituted by one or two linear or branched (C₁-C₆)alkyl groups.

2. Compound of formula (I) according to claim 1, wherein R₁, R₂, R₃, R₄, R₆, R₈, R₉ and R₁₀, which may be the same or different, each represent a group selected from hydrogen, hydroxy, linear or branched (C₁-C₆)alkoxy, linear or branched (C₁-C₆)alkyl, arylalkoxy in which the alkoxy group is linear or branched (C₁-C₆) and alkoxycarbonylalkoxy in which each of the alkoxy groups is linear or branched (C₁-C₆).

3. Compound of formula (I) according to claim 1, wherein R₁, R₂, R₃, R₄, R₆, R₈, R₉ and R₁₀, which may be the same or different, each represent a group selected from hydrogen, hydroxy, linear or branched (C₁-C₆)alkoxy, linear or branched (C₁-C₆)alkyl, arylalkoxy in which the alkoxy group is linear or branched (C₁-C₆), alkoxycarbonylalkoxy in which each of the alkoxy groups is linear or branched (C₁-C₆), and OR' in which R' represents a group selected from phosphate -PO(OH)₂, sulphate -SO₃H, carboxyalkylcarbonyl in which the alkyl group is linear or branched (C₁-C₆), dialkylaminoalkylcarbonyl in which each of the alkyl groups is linear or branched (C₁-C₆), and carboxyalkylaminocarbonyl in which the alkyl group is linear or branched (C₁-C₆).

4. Compound of formula (I) according to any one of claims 1 to 3, wherein R₅ represents an aryl group.

5. Compound of formula (I) according to any one of claims 1 to 4, wherein R₇ represents a hydrogen atom.

6. Compound of formula (I) according to any one of claims 1 to 4, wherein R₇ represents an optionally substituted nitrogen-containing heterocycle.

7. Compound of formula (I) according to any one of claims 1 to 6, wherein R₅ represents a phenyl group and R₇ represents a hydrogen atom or a substituted 1,2,3,6-tetrahydro-4-pyridyl group.

8. Compound of formula (I) according to claim 1, which is 3-(5-hydroxy-4-oxo-4*H*-1-benzopyran-2-yl)-1-phenyl-1*H* quinolin-4-one.

9. Compound of formula (I) according to claim 1, which is 3-[5,7-dimethoxy-8-(1-methyl-1,2,5,6-tetrahydropyridin-4-yl)-4-oxo-4*H*-1-benzopyran-2-yl]-1-phenyl-1*H-*1,4-dihydroquinolin-4-one.

10. Compound of formula (I) according to claim 1, which is 3-(5,7-dihydroxy-4-oxo-4H-1-benzopyran-2-yl)-1-phenyl-1*H*-1,4-dihydroquinolin-4-one.

11. Compound of formula (I) according to claim 1, which is 3-[5,7-dihydroxy-8-(1-methyl-1,2,5,6-tetrahydropyridin-4-yl)-4-oxo-4*H*-1-benzopyran-2-yl]-1-phenyl-1*H-*1,4-dihydroquinolin-4-one.

12. Process for the preparation of compounds of formula (I) according to claim 1, **characterised in that** a compound of formula (II) : 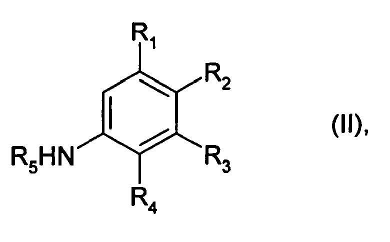 wherein R₁, R₂, R₃, R₄ and R₅ are as defined for formula (I),
is reacted with diethyl ethoxymethylenemalonate to yield the compound of formula (III) : 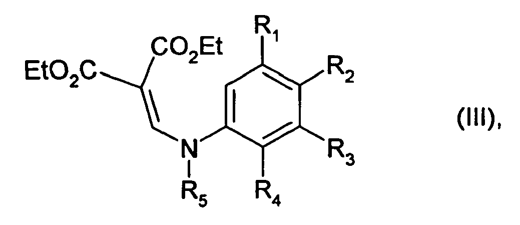 wherein R₁, R₂, R₃, R₄ and R₅ are as defined hereinbefore and Et represents an ethyl group,
which is cyclised under acid conditions to yield the compound of formula (IV) : 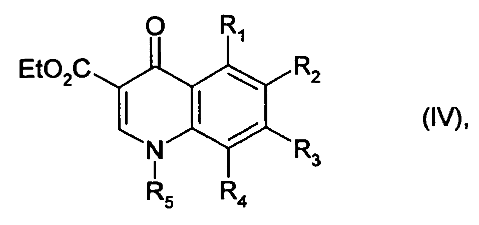 wherein R₁, R₂, R₃, R₄ and R₅ and Et are as defined hereinbefore,
which is hydrolysed to yield the compound of formula (V) : 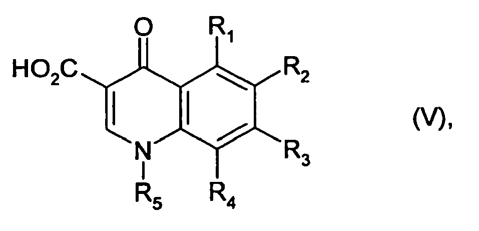 wherein R₁, R₂, R₃, R₄ and R₅ are as defined hereinbefore,
which is converted by the action of thionyl chloride into an acid chloride, which is then reacted with the compound of formula (VI) : 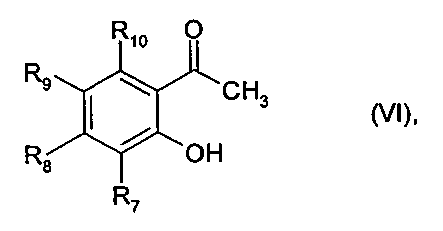 wherein R₇, R₈, R₉ and R₁₀ are as defined for formula (I),
to yield the compound of formula (VII): 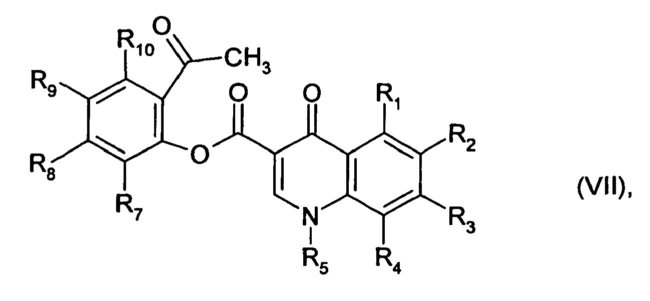 wherein R₁, R₂, R₃, R₄, R₅, R₆, R₇, R₈, R₉ and R₁₀ are as defined hereinbefore,
which is subjected to the action of a base to yield the compound of formula (VIII) : 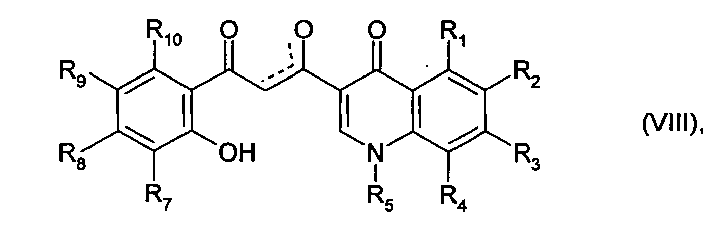 wherein R₁, R₂, R₃, R₄, R₅, R₆, R₇, R₈, R₉ and R₁₀ are as defined hereinbefore, and 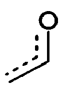 indicates that the compound is obtained, depending on the molecules involved, in the form of a keto-enol mixture,
which is then subjected to acid conditions to yield the compound of formula (I), which is purified, if necessary, according to a conventional purification technique, which is separated, if necessary, into its optical isomers according to a conventional separation technique and which is converted, if desired, into their addition salts with a pharmaceutically acceptable acid.

13. Pharmaceutical composition comprising as active ingredient a compound according to any one of claims 1 to 11, in combination with one or more inert, non-toxic, pharmaceutically acceptable carriers.

14. Pharmaceutical composition according to claim 13, for use as an anti-cancer medicament.
